# EUROPEAN PATENT SPECIFICATION

(11) **EP 0 979 656 B1**
(45) Date of publication and mention of the grant of the patent: **13.08.2008**
(21) Application number: 98830776.5
(22) Date of filing: 23.12.1998
(51) Int. Cl.: A61K 51/12, A61P 35/00

(54) **Composition containing radioisotopes immobilised on solid particles, and its use in brachytherapy**
Zusammensetzung enthaltend auf festen Teilchen immobilisierte Radioisotopen, sowie deren Verwendung in der Brachytherapie
Composition contenant des radioisotopes immobilisés sur des particules solides, et son utilisation en brachytherapie

(30) Priority: 11.05.1998 IT RM980303
(43) Date of publication of application: 16.02.2000
(73) Proprietor: ENEA ENTE PER LE NUOVE TECNOLOGIE, L'ENERGIA E L'AMBIENTE, 00196 Roma (IT)
(72) Inventor: Sedda, Antioco Franco, 00062 Bracciano (RM) (IT); Adamo, Mariana, 00062 Bracciano (RM) (IT); Rossi, Gabriele, 00069 Trevignano Romano (RM) (IT); Cipriani, Cesidio, 00185 Roma (IT)
(74) Representative: Banchetti, Marina

(56) References cited:
- EP-A- 0 030 735
- WO-A-97/49335
- US-A- 5 061 475
- US-A- 5 362 473
- MUMPER R J ET AL: "NEUTRON-ACTIVATED HOLMIUM-166-POLY (L-LACTIC ACID) MICROSPHERES: A POTENTIAL AGENT FOR THE INTERNAL RADIATION THERAPY OF HEPATIC TUMORS" JOURNAL OF NUCLEAR MEDICINE, SOCIETY OF NUCLEAR MEDICINE. NEW YORK, US, vol. 32, no. 11, 1 November 1991 (1991-11-01), pages 2139-2143, XP000240389 ISSN: 0161-5505
- PATENT ABSTRACTS OF JAPAN vol. 0185, no. 88 (C-1271), 10 November 1994 (1994-11-10) & JP 6 218276 A (MITSUI TOATSU CHEM INC), 9 August 1994 (1994-08-09)
- MARYANSKI M J ET AL: "RADIATION THERAPY DOSIMETRY USING MAGNETIC RESONANCE IMAGING OF POLYMER GELS" MEDICAL PHYSICS, AMERICAN INSTITUTE OF PHYSICS. NEW YORK, US, vol. 23, no. 5, 1 May 1996 (1996-05-01), pages 699-705, XP000592654 ISSN: 0094-2405
- AKUDUGU J M ET AL: "In vitro soft agar model for experimental brachytherapy: Absorbed dose measurements" ENDOCURIETHERAPY/HYPERTHERMIA ONCOLOGY 1996 UNITED STATES, vol. 12, no. 1, 1996, pages 51-62, XP0008013965 ISSN: 8756-1689

## Description

The present invention concerns a composition containing radioisotopes immobilized on solid particles, useful in particular for the clinical brachytherapy of tumoral diseases. More specifically, the invention relates to a composite preparation particularly suited for the therapy of cancer by means of the application of radioactive sources in direct contact with the tumor tissues or within the same, known as brachytherapy. The preparation is also useful for use in other medical techniques employing the intracavitary, intratissutal (interstitial) or intraluminal (intraarterial) application of radioactive microparticles.

As it is known, there are many forms of cancer against which the conventional treatments, substantially based on surgery combined with external radiotherapy and/or with chemotherapy, are often ineffective. In other cases the conventional treatments are scarcely applicable in view of the location and diffusion of the lesions, or in view of the general conditions of the patient. Among such forms or cancer, brain tumors and liver tumors, both primary and originated from metastases, are particularly critical.

Brain tumors, in particular, have a high grade of mortality and are only occasionally treated successfully. In adults, some brain tumors are actually metastatic lesions from cancer of the breast, of the lung, of the intestine or from malignant melanoma. However, brain tumors are most frequently primary tumors, and among these the most widespread ones are the tumors belonging to the group of gliomas, such as astrocytoma, oligodendroglioma and glioblastoma multiforme. Such tumors essentially consist in the neoplastic degeneration of the glial cells, which represent 90% of the central nervous system, and serve as a support for neural cells. Contrary to the neural cells, glial cells undergo birth, differentiation and mitosis cycles, and this renders such cells much more prone to the risk of tumoral degeneration than neural cells.

The most lethal of the brain cancer forms cited above is glioblastoma multiforme (also known as anaplastic astrocytoma), a rapidly growing infiltrating tumor, that develops within the cerebral mass with branched projections similar to pseudopods. Glioblastoma multiforme is responsible, in the only United States of America, of more than 6000 deaths per year, with a markedly increasing trend. As the other brain tumors, glioblastoma is treated first of all with surgery, in every case where this is possible. However, local recurrence is very frequent, owing to the practical impossibility of eliminating, even by a surgical exeresis macroscopically appearing quite radical, all of the tumor cells infiltrated in the surrounding healthy tissues.

As a matter of fact, one of the most frequent mechanisms that may result in the recurrence of a neoplastic formation is regrowth by contiguity. The regrowth is started by even extremely few tumor cells left in proximity of the excised tumoral mass. These cells, by rapidly multiplying, reinstate in a short time the pathological condition. Actually, it has been reported that about 90% of the recurrences of glioblastoma multiforme are located within a margin of no more than 2 cm from the original tumor site. In order to reduce such occurrence the treatment protocols for glioblastoma normally include, as it normally happens in the therapy of other neoplastic diseases, a postoperative treatment by radiotherapy. Theoretically, the cancerous cells of gliomas are prone to the lethal action of radiation as are all cells having reproductive cycles. At the same time, advantage may be taken from the fact that, differently from other types of cells (such as, e.g., germinal cells), all neural cells, including the cells of the healthy tissues surrounding the tumor site (i.e., neurones, glial cells), are particularly resistant to radiation.

After the surgery, therefore, the patients undergo one or more cycles of irradiation of the tissue surrounding the original tumor site with electron beams or with gamma radiation. Such therapeutic approach, however, even when applied with the most sophisticated techniques, such as the use of lamellar collimators, fractionated stereotactic radiotherapy or the use of radio-sensitizing agents, has shown some efficacy only in the lowest grade forms. In fact, quite seldom external radiotherapy may be applied at the doses that are theoretically necessary to obtain a complete eradication of the tumor cells, owing to the concurrent damage produced on the healthy neural tissue. On the other hand, chemotherapy does not represent a suitable alternative resource, since the blood-brain barrier hinders or reduces the effect of many anticancer agents administered by the systemic route.

Depending on the grade, the extension of the lesions and the treatments applied, therefore, the survival for patients with glioblastoma multiforme is presently comprised between a few months and about two years. Even with the most advanced therapy protocols the life expectancy after a high-grade diagnosis remains of ten months at the most.

In order to limit as much as possible the damage induced by the irradiation on the healthy tissues, the postoperative treatment of brain tumors may resort to administration techniques that use the local-regional route, exploiting the principles of brachytherapy. A particular reservoir is used for such purpose, known as the Ommaya reservoir, which is permanently placed close to the brain lesion, after the surgical excision. The Ommaya reservoir substantially consists of a small silicone bag ending in a tube with a radio-opaque terminal having a plurality of holes. The bag is placed under the patient's scalp, so as to be accessible through a simple hypodermic injection, while the perforated tip is inserted in the brain cavity left by the excised tumoral mass. Such system is presently used to introduce in a periodically repeated and non-invasive way chemotherapeutic agents or also labelled monoclonal antibodies into the brain. The said agents are injected in the bag under the skin and then, by a simple pressure of the fingers, they are made to penetrate the brain cavity through the perforated tip.

A known therapeutic protocol employing the Ommaya reservoir for the intracavitary administration of radioactive sources uses monoclonal antibodies reactive with antigens typical of the external matrix of tumor cells (specifically, anti-tenascin antibodies) labelled with radioactive iodine. The antibodies administered in the surgical cavity are deemed to selectively bind the tumoral cells still present, thus exerting the lethal activity of the radioisotope in a targeted manner. Irrespective of the actual capacity of the antibodies to bind the glioblastoma cells, on which contradictory evidences exist at present, the concerned solution has in any case the effect of performing a local radiotherapy, irradiating the tissue adjacent to the margin of the surgical excision, without using any external beams.

In order to perform a valid brachytherapy by intracavitary application of radioactive sources (and exploiting, in the case of glioblastoma multiforme as in other brain tumors, the use of the Ommaya reservoir) it is extremely important that the internal surface of the cavity created by the surgeon with the excision of the tumoral tissue (that only initially has an approximately spherical shape, but soon folds on itself, collapsing and forming furrows and interstices) is irradiated as uniformly as possible. A layer of tissue some millimetres thick around the cavity has to be selectively irradiated without appreciably damaging, at the same time, the surrounding tissue. To that aim, the introduction of a liquid agent would be ineffective (unless such liquid was provided with means for selectively and reliably binding some given tumor antigens), as in a very short time the liquid would diffuse through the tissues, dispersing and indiscriminately irradiating wide zones of the body.

On the other hand, neither a source consisting of one or more solid elements of macroscopic size, such as the needles or capsules made of a radioactive material (e.g., iridium-192) well-known in brachytherapy for intrauterine, intrarectal or intraesophageal application, could be used in the case at issue. Actually, not only it would be practically impossible, in this case, to place the sources within the concerned tissue in such a way to allow their easy renovation, but, in addition, the irradiation obtained on the internal walls of the irregularly shaped cavity would not be uniform.

A different alternative, already experimented in other fields, such as the treatment of arthritis with injections, into the affected joint, of a suspension containing holmium-166 in a particulate carrier, as disclosed in US 5061475, is to employ solid particles non diffusible in the tissues (that would remain confined within the surgical cavity), such as microspheres made of resin, glass or other biocompatible material, loaded with the suitable radioisotopes and injected in the site of action in the form of suspensions in a liquid medium. An example of the use of such particles is disclosed by Tian et al. (Jia-He Tian, Bai-Xuan Xu, Jin-Ming Zhang, Bao-Wei Dong, Ping Liang and Xiang-Dong Wang, J. Nucl. Med., 37(6), 958-963, (1996)). These authors propose the use of glass microspheres loaded with yttrium-90 for the treatment (brachytherapy) of liver malignancies, by intratissutal injection into the tumor. In the above publication the authors consider the interstitial application of the radioactive microspheres as an alternative to the already experimented solution consisting in the introduction of such microspheres, close to the tumor, by intraarterial infusion, through catheterisation of the hepatic artery. According to the cited document, the glass microspheres loaded with ⁹⁰Y, a high energy beta-emitting radioisotope particularly suitable for brachytherapy, are introduced in the tumor tissue suspended in iodinated oil.

Another solution employing microspheres carrying a radioisotope (also in this case, yttrium-90) allows to overcome the drawback typical of glass microspheres (or of resin microspheres, that are also known and used in this field) of not being bioabsorbable. Such solution is disclosed, *inter alia*, by Häfeli et al. (Urs O. Häfeli, Siobhan M. Sweeney, Beverly A. Beresford, Edward H. Sim e Roger M. Macklis, J. Biomed. Mat. Res, 28 901-908, (1994)). The microspheres disclosed are made of poly(lactic acid), a polymer of lactic acid having a molecular weight in the range from 1000 to 60,000, that may be metabolised by the human body according to the normal metabolic routes up to lactic acid, the latter being a common physiological constituent. Polylactic acid contains carboxyl groups that are able to chelate metal cations (in particular, the trivalent yttrium cations) in the same way as any synthetic ion exchange resin, and therefore it is proposed for the production of radioisotope-carrying microspheres for various applications, both diagnostic and therapeutic.

The composition of poly(lactic acid) ⁹⁰Y-microspheres described in the above document also includes a remarkable amount of magnetite (Fe₃O₄), with the aim of making the particles reactive to an external magnetic field. This is intended to be of use in guiding the microspheres, once introduced within the body, towards the desired site of action. The said publication, however, only concerns a phase of *in vitro* experimentation, and does not deal with the applicative aspect of a possible clinical procedure based on the use of the proposed microspheres. In the experimentation reported, the polylactic acid microspheres are suspended in a liquid medium.

With specific reference to the application for brachytherapy of brain tumors by intracavitary administration considered herein, it is evident that the use of radioactive microparticles suspended in a liquid medium as described in the foregoing documents does not solve the problem of providing a uniform dose deposition within the surgical cavity, since the particles would tend to settle by gravity, thus preferentially irradiating the lowest part of the cavity. The non-uniform distribution of the microsources is particularly critical if one considers that the radioisotopes employed for this type of applications are beta-emitters, i.e. electrons. Contrary to gamma-emitters, beta-emitters have a useful emission range not exceeding 1 cm. Such low penetration capacity, while being required in order to avoid an excessive damage to the healthy tissue close to the lesion, requires, on the other hand, a closer contact between the tissue to be treated and the radioactive sources.

The problem of a correct positioning of the radioactive sources in carrying out brachytherapy methods is dealt with, yet with no specific reference to glioblastoma, in several patent documents. EP-A-0 292 630 (Horowitz) discloses the use of a bioabsorbable solid support structure incorporating, regularly spaced from each other, radioactive seeds containing gamma-emitting isotopes. The structure, although allowing a uniform distribution of the sources, cannot result in a really homogeneous radiation on every interstice of a relatively small cavity of a complex shape, owing to the macroscopic size of each seed. In addition, the said structure is not suitable for implantation within the brain, nor it is suitable for use with beta-emitting radioisotopes, owing to self-shielding problems.

WO-A-97 49335 (DuPont Merck) discloses the use of an insoluble phosphorus salt (³²P, a beta-emitter) combined with a pharmaceutical vehicle and optionally with a thickening agent, for the administration as an aqueous suspension (possibly with the addition of an inert particulate support material, such as carbon particles) by direct injection into the solid tumor (intratissutal treatment). As an alternative, the composition may be introduced into polymeric matrices in the form of thin needles, to be implanted by injection. Also in this case, the solutions suggested do not solve the problem referred to in the foregoing of achieving a uniform dose distribution, especially in the case of intracavitary administration.

The said problem is neither solved by the teachings of WO-A-97 19706 (Coniglione), concerning solid sources consisting of a dispersion of radioactive powders, beta- and gamma-emitting, in solid biocompatible (and, preferably, bioabsorbable) polymeric matrices. Such sources may have a wide range of shapes, including solid or hollow needles, sutures or threads, films or sheets, and also microspheres. In the latter case, however, the method of administration of the microspheres to the site to be treated is not disclosed.

Within the frame of radioactive particulate compositions for use in the medical field, US 5362473 discloses a composition for use in lung scintigraphy, comprising radioactive labelled particles on a base of silica gel, or similar materials, to be administered in the form of a dry aerosol.

Therefore, it is an object of the present invention to provide a radiopharmaceutical product particularly suitable for the intracavitary therapy of neoplastic diseases, which is capable of irradiating in a uniform and selective way the interior of a cavity such as that resulting from the surgical removal of tumoral masses, reaching with its radiation all of the interstices that may be present within such a cavity and being gradually absorbed after some time, while not dispersing the radioactive agents in the surrounding tissues.

To achieve such purpose, the present invention proposes to suspend in a specifically designed medium radioactive microparticles of the same kind as those described in the prior art, consisting of biocompatible (and, preferably, bioabsorbable) solid particulate carriers on which radioisotopes, preferably beta-emitters, are immobilized. Such medium, consisting of a gel containing polyvinylpyrrolidone and agar, in addition to being biocompatible and bioabsorbable, is also such as to hold the microparticles in stable suspension at the body temperature, having at such temperature the consistency of a jelly. The gel proposed has also the characteristic of reversibly loosing most of its viscosity when heated to temperatures of about 70-80°C, so that it can be easily mixed with the radioactive particles and administered to the patient by means of a syringe, and it can readily and homogeneously fill up the concerned cavity. Then, the gel can revert to a higher thickness so as to stably maintain the particles in a dispersed state in the cavity. In addition, the proposed gel has such a composition, as a result of the molar concentration of its components, to be hypotonic. This results in the water contained therein tending to gradually diffuse in the surrounding tissue, thus bringing the suspended radioactive particles, that remain trapped in the cavity, closer to the margins to be irradiated as the thickness of the cavity decreases. In this way most radioactive particles employed can strike with their emission (although such emission is not highly penetrating) the tissue surrounding the cavity, thus providing a high absorbed dose of radiation in a quite limited area.

Furthermore, in the event that, in accordance with a preferred embodiment of the invention, the solid microparticles are made of a bioabsorbable material such as the poly(lactic acid) referred to in the foregoing, after some time from the therapy no trace of foreign bodies is left within the cavity.

Although it has been specifically designed for brachytherapy by intracavitary administration, the preparation according to the invention is also suited for interstitial administration by injection into the tumor tissue, by a technique such as that disclosed by Tian et al. (*loc. cit.*). In this case the uniform distribution of the radioactive particles suspended within the gel results in a good control of the dosage administered with the injection. Further, the preparation is also suited for intraarterial administration through a catheter. In this case, the preferred solution using the gel in combination with bioabsorbable particles is even more advantageous, in view of the fact that such solution allows to avoid the irreversible obstruction of the capillary vessels in which the microparticles stop when carried by the bloodstream.

Therefore, the preparation according to the present invention may be employed in the treatment by brachytherapy of several tumoral diseases, either malignant or not. As it will be shown further on, the said diseases include not only brain tumors - and, in particular, glioblastoma multiforme - but also, for instance, primary liver tumors and hepatic metastases, as well as hypophysis adenomas. In addition, the composition according to the invention may be employed in other medical techniques not related to the treatment of neoplastic affections, in all cases where a local-regional application of radioactive microsources may be useful, e.g. for the purpose of obtaining a reduction in the functionality and volume of the spleen by radioembolization, in patients with hypersplenism of various origin, Further, the composition may be applied as an aid in radio-guided surgery, in particular it may be injected in tumoral lesions of the breast during the examination preceding the surgical operation, in place of the currently used "flagging" system for breast tumoral nodules, consisting in the introduction of metallic wire bits in the said nodules.

Accordingly, the present invention specifically provides a radioactive composition for use in therapy and as a surgical aid containing radioisotopes immobilized on biocompatible or bioabsorbable solid particles obtainable from separately produced solid particles, on which the said radioisotopes are subsequently immobilized by chelation, or obtainable from particles that already incorporate the non-radioactive chemical element corresponding to the desired radioisotope and which particles are then irradiated in a nuclear reactor under neutron flux to obtain the said radioisotopes, characterised in that the said solid particles are incorporated in a biocompatible and bioabsorbable matrix consisting of a hypotonic gel containing, in water, from 2 to 30% by weight of polyvinylpyrrolidone and from 0.01 to 2% by weight of agar-agar or agarose. Preferably, in order to achieve the desired value of osmolarity, the average molecular weight of polyvinylpyrrolidone is in the range from 50,000 to 400,000.

As it is known, polyvinylpyrrolidone (PVP) is a polyvinyl polymer of N-vinyl-2-pyrrolidone, having molecular weight ranging from 1000 and 700,000 or more, obtained by free radical polymerisation of vinyl pyrrolidone in water or in isopropanol. In its straight chain form (also known as "povidone"), polyvinylpyrrolidone is readily soluble in water, and is also soluble in several organic solvents. PVP is widely used in the pharmaceutical art as a dispersing and suspending agent, and it has also been employed for a long time as a plasma substitute in traumatic or hemorrhagic shock, in presence of defective peripheral blood circulation.

PVP is described, e.g., in the corresponding monographs "Polyvidonum" and "Povidone" of the latest editions of the European Pharmacopoeia, of the Italian Pharmacopoeia, of USP 23 etc.. In these Pharmacopoeias the molecular weight of soluble PVP is expressed in terms of the value of a K parameter which is calculated from the relative viscosity in water and often forms part of the commercial name. Obviously, each product consists of a mixture wherein various proportions of molecules with different molecular weights are present. In the ideal case, such molecular weights have a gaussian distribution. These mixtures, which may be evidenced and characterised with the help of the gel-permeation chromatography, may contain, normally, up to a maximum of 15-20% of lower molecular weight fractions and about 20% of fractions of higher molecular weight.

The aqueous solutions of PVP may be sterilised in autoclave (15-20 min. at 120°C) with no modifications of their molecular weight as a result of this treatment. In addition, it is important to consider that trials on man and on animals have shown that PVP has no carcinogenicity (IARC Monograph, Suppl. 7 (1987), 73).

Agar-agar (also known as agar, or gelose) is a natural polysaccharide extracted from various species of algae, widely employed as an additive in foods and medicaments for its thickening properties. Agar substantially consists of a fraction (normally amounting to 55-66% by weight) of agarose, a neutral, linear polysaccharide which is responsible for the gelling properties of agar, and a residual fraction of another linear polysaccharide having ionisable groups, agaropectin. For most uses in the food industry and in the pharmaceutical field, agar is employed without any previous separation of agarose from agaropectin, while the use of agarose in purified form is normally limited to laboratory techniques such as chromatographic separations and enzyme immobilization.

Agar-agar has been tested for carcinogenicity and long term effects by the U.S. National Program for years, and it has been found to be non-carcinogenic (National Toxicology Program Research & Testing Division 1992, Report No. TR-230, NIEHS, Research Triangle park, N.C., USA).

Obviously, the preparation of the gel according to the invention will require the use of high purity products, besides sterilised and apyrogen deionised water. It should be appreciated that other possible gel-forming products, different from the proposed agar-PVP combination, cannot be used for the purposes of the present invention, either because they do not have the required physico-chemical characteristics described above, or for other practical reasons, such as toxicity problems or high cost. In particular, the use of bovine collagen is to be excluded due to the possibility of transmission of bovine spongiform encephalopaty, and the use of human collagen is to be excluded for obvious reasons. The use of hyaluronic acid would be quite expensive and, moreover, this agent is suspected of stimulating the growth and migration of glioblastoma cells (Shravan et al., Frontiers in Bioscience, 1, d324-329, 1.11.1996). Pectins and carrageenans, that are other naturally occurring polysaccharides employed in the food industry and in the pharmaceutical field, do not show the required rheologic properties and, in addition, they interfere with the immune system, deactivating the macrophages.

According to a preferred embodiment of the invention, the proposed gel has the following formulation (the percentages being by weight):
- PVP with average MW 340,000-380,000 15-25%
- purified agar-agar 0.1-0.2%
- deionised sterile water q.s. to 100%.

In general, the gel may be prepared by dosing the components of the formulation in relative proportions variable within the ranges set forth above, such proportions being such as to obtain, at high temperature, a suitable fluidity to allow the subsequent mixing with the radioisotope-carrying particles and the flowability of the preparation in the syringe needle. At the same time, the said proportions should be such as to result, at the body temperature, in sufficiently high viscosity and density to keep the particles in a stable suspension. The components are dissolved in hot water while stirring, until they form a perfectly clear solution, which is then autoclaved at 121°C for sterilisation, preferably in glass vessels. The vessels are then hermetically sealed and stored at 4°C.

The second component of the product according to the invention, namely the biocompatible or bioabsorbable solid microparticles loaded with a radioisotope, may be prepared according to known general methods, either starting from separately produced solid particles, e.g. made of a suitable ion exchange resin or of poly(lactic acid) on which the radioisotope is subsequently immobilized by chelation, or starting from particles that already incorporate the non-radioactive chemical element corresponding to the desired radioisotope, which particles are then irradiated in a nuclear reactor under neutron flux to obtain the corresponding active isotope. Such technique is disclosed, in connection with poly(lactic acid) microspheres and to the non-radioactive element holmium-165, by Mumper et al. (R.J. Mumper, U. Yun Ryo e M. Jay, "Neutron activated Holmium-166-poly(l-lactic) microspheres: a potential agent for the internal radiotherapy of hepatic tumours", J. Nucl. Med., 32, 2139-2143 (1991)).

Irrespective of the constituent material, in order to be validly employed in the various applications according to the invention, the concerned solid particles should have diameters in the range from 0.2 µm to 100 µm. In particular, while for the use in brachytherapy by intracavitary and intratissutal administration the particle size is not particularly critical (such size being, in the most common cases, between 20 and 50 µm), for the application by the intraarterial route (e.g., in the treatment of liver tumors or in the spleen volume reduction by radioembolization) such size should be such that the particles may pass through the capillaries and stop in the finest ones, thus offering an optimal distribution of the radioisotope in the tissue, without passing beyond the target organ. Accordingly, in such cases the most suitable size is in the range of 15-30 µm. In other applications, particularly designed for polylactic acid, the concerned particles are coated with polylysine, polyomithine or with amino acids containing an arginine-lysine-aspartic acid group, in order to render such particles adhesive to the treated tissues. In this case, the preferred sizes of the particles for use in applications according to the invention are sub-micronic.

According to some specific embodiments of the invention, the microparticles are made of a biocompatible anion or cation exchange resin, e.g. a styrene-divinylbenzene resin with ammonium chloride type anionic exchange groups (such as the AG1x8 resin by Dowex), or a biocompatible acrylic resin with carboxyl exchange groups such as the one marketed under the trade name Bio Rex 70. An example of biocompatible (and potentially biodegradable) acrylic resins for the production of microcapsules and microspheres for pharmaceutical use - i.e. poly(alkyl-2-cyanoacrylates) - is disclosed, e.g., by Florence et al. (AT. Florence, T.L. Whateley, D.A. Wood, J. Pharm. Pharmacol., Communications 31, 422-423, (1979)). In the preparation of the resin for use in the composition according to the present invention, the fixed amount of resin (typically, 30-150 mg for one application, but this amount may vary in accordance with the therapeutic use of the composition and to the relevant protocol) is weighed in the dry state and is then placed in a container, preferably made of glass, together with an equal volume of deionised water. The mixture is then sterilised in autoclave at 121°C, and is then hermetically sealed and stored at 4°C. The storage in the form of an aqueous mixture is preferred in order to stabilise beforehand the resin that, by its own nature, is subject to swelling.

According to another embodiment of the invention, that is presently preferred, the microparticles are made of poly(lactic acid), and are obtained according to a method such as that disclosed in the publication by Häfeli et al. referred to in the foregoing. Since poly(lactic acid) is a solid material insoluble in water but soluble in organic solvents, if an organic solution of such compound is very rapidly injected into an aqueous solution containing polyvinyl alcohol (PVA) and is subjected to vigorous stirring, an emulsion is formed which, upon the gradual passage of organic solvent in the aqueous phase, forms a precipitate of spherical particles of poly(lactic acid). Specifically, according to the procedure that may be used for the purpose of the present invention, 10-100 mg of acid poly(lactic acid) having a preferred molecular weight of 2000 is dissolved in 1-5 ml of methylene chloride together with 1-20 µg of L-α-phosphatidilcholine (serving as an emulsion adjuvant) and, optionally, 1-10 mg of Fe₃O₄ powder with particle size < 1 µm. The iron oxide (magnetite) particles are incorporated within the poly(lactic acid) microspheres in order to make the latter X-ray-opaque, and are bioabsorbable by the human body as is poly(lactic acid). The mixture is sonicated for some seconds to form a homogeneous suspension, and the suspension so obtained is rapidly injected in at least 50 ml of an aqueous solution (containing water alone or with sodium phosphate buffer) containing 1-5% by weight PVA with a MW in the range from 70,000 to 120,000. The mixture is kept under rapid stirring for some time. The microspheres thus formed are the separated by filtration, washed and dried. Depending on the molecular weight of polyvinyl alcohol, on the concentration thereof and on the stirring speed spherical particles of poly(lactic acid) with diameters comprised between 0.01 µm and 100 µm are obtained.

In a variant of the preparative process described above, as pointed out, before the poly(lactic acid) microspheres may be produced including in the starting mixture a complex, soluble in organic solvents, of the chemical non-radioactive element corresponding to the radioisotope desired in the final radiopharmaceutical product, such as, e.g., ¹⁶⁵Ho-acetylacetonate, as described by Mumper et al. (*loc. cit.*). In this way, the polylactic acid microspheres will directly incorporate, in a dispersed form, the element that will originate the active isotope (for instance, ¹⁶⁶Ho) upon irradiation in a nuclear reactor.

When poly(lactic acid) is used, in a preliminary preparation stage of the composition according to the invention the fixed amount of microspheres with the desired particle size (also in this case, a typical amount being 30-150 mg per each application), obtained as above, is washed in deionised water, placed in a sealed glass container and sterilised by gamma photon radiation. In this case, the microspheres are stored in the dry state.

The isotopes useful for the purpose of the invention are in general beta- and/or gamma-emitting radioisotopes, generally with energy > 0,3 MeV. Particularly suited for brachytherapy applications are beta-emitters, which allow to selectively irradiate a few millimetres only of tissue, with a high delivered dose, without appreciably damaging the neighbouring tissue. On the other hand, for the application of the product as a "flag" in radio-guided surgery the gamma-emitting radioisotopes are preferred, in view of their high penetrating capacity. Particularly interesting are the ⁹⁰Y, ¹⁶⁶Ho, ¹³¹I, ¹⁸⁶Re and ¹⁸⁸Re isotopes, either produced in nuclear reactor by irradiation of non-radioactive elements with neutron flux, or obtained from suitable "isotope generators". The latter are relatively long-lived radioactive precursors that generate by decay the concerned isotopes, that are progressively separated from the generator element. An example of such system is the one starting from ⁹⁰Sr, a nuclear fission by-product, and leading by decay to the formation of ⁹⁰Y.

Among the above-mentioned elements, iodine-131 (obtainable, e.g., by fission from ²³⁶U, through irradiation in a reactor) is widely used in radiotherapy, in particular for the treatment of thyroid gland. ¹³¹I is characterised by a half-life of 8 days and both beta- and gamma-emission. Such element is particularly suited for the production of compositions according to the invention for use as "flag" in radio-guided surgery, as it will be explained further on. Yttrium-90, as pointed out before, is a pure beta-emitting radioisotope, with a half-life of 64 hours and a maximum energy of 2.3 MeV, and is presently preferred in brachytherapy applications in view of the high energy and the low penetration or its emission. Another radioisotope recently employed in this field is holmium-166, already mentioned above, that is a beta-emitter having about 5% of gamma-emission at 81 keV. This feature makes ¹⁶⁶Ho valuable for use in the visualisation by conventional scintigraphy in the course of therapy. The maximum energy of holmium-166 is 1.9 MeV and its half-life is about 26.5 hours, this feature making it less practical for use. However, holmium-166 has the advantage of having an activation section about 60 times greater than that of yttrium-90, and for this reason it may be used in the preparations in amounts up to 60 times smaller.

The active isotopes useful for the composition according to the invention may be obtained, as noted, by radiochemical separation from generators, in which case they will be in the "carrier-free" form (i.e., with all atoms of the same chemical species being radioactive), or by irradiation in reactor, in which case the ratio between active and non-active atoms may be of the order of 10⁴-10⁵. If all activated atoms are of the same chemical species as the starting atoms (such as, e.g., in the case of activation of ⁸⁹Y to ⁹⁰Y) the product is obtained in the "carrier-added" form; it on the contrary (as in the case of production of ¹³¹I from ²³⁵U), the resulting element is chemically different from the starting element, the separation is necessary, and the final product will be of the "carrier-free" type.

In the embodiments of the invention wherein the radioisotope is produced separately from the solid support microparticles, a ionic compound comprising the element corresponding to the desired radioisotope, e.g. yttrium nitrate, is irradiated in a reactor, for instance with a neutron flux of 10¹²-10¹³ n cm⁻² s⁻¹, for 2-4 hours. Then, the said compound is placed in a suitable buffer, and the solution is sterilised by filtration. Preferably, the amount of solution is the minimum amount that is capable of wetting the fixed amount of particles with which the solution is to be contacted, and the amount of radioisotope contained therein does not exceed the maximum loading capacity of the resin or of the poly(lactic acid). The level of radioisotope activity is the one that is found, on the basis of dosimetric calculations with suitable theoretical models, to give the desired absorbed dose at the various depths in the exposed tissue.

The solution is then contacted with the biocompatible or bioabsorbable microparticles and stirred for a period of time (normally about 1 hour) sufficient to achieve a complete chelation reaction between the resin or the poly(lactic acid) and the radioisotope. The reaction is carried out at room temperature when a resin is used and at 37°C when polylactic acid is used. Considering the resin particles, it has been found that styrene-divynilbenzene ionic exchange resins of the type of AG1x8 are more suitable for chelation of iodine, while for the other possible cations the best results in terms of stability and loading capacity are obtained with acrylic resins of the type of Bio Rex 70. Both when using biocompatible resins such as those cited above and when using poly(lactic acid), the stability of chelation was always found to be satisfactory. The particles thus obtained (after a proper washing and removal of any possible residual amount of unbound radioisotope) were practically not leachable. Stability tests on such microparticles in human serum at 37°C have shown losses of no more than 0.2-2% of the initial activity in 24-48 hours.

After chelation, the liquid is removed and, preferably, the particles are washed with a sterile solution and mixed with the gel according to the invention. The latter has been heated meanwhile to 70-80°C for some minutes so as to fully liquefy. After a vigorous stirring, such as to give homogeneous suspension, the composition according to the invention is ready for administration by injection as prescribed by the medical specialist.

In the embodiments of the invention wherein the element corresponding to the desired radioisotope is already incorporated in the microspheres, the fixed amount of microspheres (established, also in this case, on the basis of the specific therapeutic procedure in which the composition is to be used) is irradiated in a reactor as disclosed above, for a period of time sufficient to achieve the desired radioactivity in the particles. Then the microspheres are mixed with the gel of the invention already heated to 70-80°C, as described above.

The quantitative ratio between the gel and the particles loaded with the radioactive isotope may vary within a quite broad range, in accordance with the kind of pathology to be treated, the route of administration (i.e. intracavitary, intratissutal or intraluminal) and, in the case of intracavitary application, with the cavity size. In general, the said ratio is such as to provide an absolute radioactivity concentration the range from 0.1 mCi/ml to 200 mCi/ml. The possible values of the total amount of gel are comprised between 0.1 ml and 10-30 ml, and the total amount of microparticles may vary from 0.1 mg to 1-2 g.

The composition according to the invention may be made available in the form of a kit, wherein the single components are packaged in a sterile form, ready for being used upon combination according to the procedure described above, just before the injection to the patient. Therefore, the present invention further provides a radiopharmaceutical kit or a kit for use in radio-guided surgery comprising a first component containing biocompatible or bioabsorbable solid particles capable of carrying radioisotopes in a substantially not leachable manner and, specifically, chosen among bioinert particles made of ion exchange resins and bioabsorbable particles made of poly(lactic acid), and a second component consisting of a hypotonic gel containing, in water, from 2 to 30% by weight of polyvinylpyrrolidone and from 0.01 to 2% by weight of agar-agar or agarose, as described in the foregoing.

The particles of the first component may already incorporate the non-radioactive chemical element corresponding to the desired radioisotope, to be activated by irradiation in a reactor. In this case, the kit necessarily consists of two only components. In the alternative, i.e. when the microparticles do not incorporate the radioactive precursor, a third component may also be present, containing the beta- and/or gamma-emitting radioisotopes. Further features of the kit according to the invention are specified in the further dependent claims.

Some specific embodiments of the invention are described below for merely illustrative purposes, together with the results of the experimental studies carried out on the proposed compositions, in connection with some therapeutic applications of the same.

### EXAMPLE 1

A kit for use in clinical brachytherapy for the treatment of glioblastoma multiforme consisted of two glass test-tubes. One test-tube contained 5 ml of the gel according to the invention, with 20% by weight of PVP of MW 360,000 and 0.1 % by weight of ultrapure agar-agar in deionised sterile and apyrogen water. The other test-tube contained 120 mg of Bio Rex 70 resin and 0.15 ml of water. The contents of both test-tubes were sterile, as a result of a treatment in autoclave at 121°C for 15 minutes.

The radioisotope necessary for preparing the composition according to the invention was produced by irradiating 150 mg of yttrium nitrate tetrahydrate in a TRIGA reactor (by General Atomics) for 3 hours at a flux of 1,2·10¹³ n cm⁻² s⁻¹, obtaining an activity of about 2.2 mCi. The starting material employed had a chemical purity of 99.99%, and after irradiation the activity measured with a Ge detector for gamma-rays and with liquid scintillation for beta-rays was due for 99.8% to the only ⁹⁰Y. The radioactive compound (Y(NO)₃) thus obtained was placed in solution in 1 ml of 0.1 M ammonium acetate buffer, and then sterilised by filtration.

The method of use of the above kit is as follows:
a) the ⁹⁰Y solution described above is introduced, by means of a sterile syringe equipped with a 0.22 µm filter, in the test-tube containing the resin, and the mixture is kept under stirring for about 1 hour at room temperature;
b) the liquid is then sucked from the test-tube by means of a sterile syringe, and is discarded (as shown by preliminary tests, the ⁹⁰Y activity released in the liquid is approximately 3% of the initial activity);
c) meanwhile, the test-tube containing the gel is heated to 70-80°C in a water bath, so as to liquefy its contents (typically, heating is applied for 5 min.);
d) the liquid gel is taken from the test-tube by means of a sterile syringe, and is poured into the test-tube containing the resin loaded with the radioisotope, vigorously stirring until a homogeneous suspension is obtained;
e) the suspension is sucked by means of sterile syringe equipped with a 19-20 G needle, the gel is left to cool down for some minutes and is injected as prescribed (typically, by hypodermic injection in the Ommaya reservoir placed under the patient's scalp).

### EXAMPLE 2

A different kit, also for use in the intracavitary therapy of glioblastoma multiforme, consisted of two test-tubes. One test-tube contained 5 ml of the gel according to the invention, with 15% by weight of PVP of MW 360,000 and 0.2% by weight of ultrapure agar-agar in deionised sterile and apyrogen water. The other test-tube contained 34 mg of poly(lactic acid)-based microspheres, with 30 mg of poly(lactic acid) and 4 mg of holmium acetylacetonate. The contents of both test-tubes was sterile, the sterilisation having been obtained for the first test-tube by treatment in autoclave at 121°C for 15 minutes and for the second test-tube by gamma-irradiation.

To produce the composition according to the invention, the contents of the second test-tube was irradiated in a TRIGA reactor for 2 hours at a flux of 1,25·10¹³ n cm⁻² s⁻¹, obtaining a total activity of about 4 mCi, as measured by a NaI detector vs. a standard of ¹³³Ba. The test-tube contents was then sterilised by gamma-irradiation.

The method of use of the above kit is as follows:
a) the test-tube containing the gel is heated to 70-80°C in a water bath, so as to liquefy its contents (typically, heating is applied for 5 min.);
b) the poly(lactic acid) microspheres are taken from the second test-tube with 1 ml of deionised sterile and apyrogen water, by means of a sterile syringe;
c) the microspheres are poured into the liquid gel, vigorously stirring until a homogeneous suspension is obtained;
d) the suspension is sucked by means of sterile syringe equipped with a 19-20 G needle, and the procedure is continued as in item e) of the previous example.

By using compositions of the kind described in Examples 1 and 2, patients with II and III grade glioblastoma multiforme were treated, visualising the introduction of the microparticle-containing gel in the brain cavity by computed tomography (CT). It has thus been ascertained that the introduced substance forms, at the outset, an approximately spherical deposit, and that the gel is later gradually absorbed in the surrounding tissue, thus leaving the dispersed radioactive particles in contact with all of the interstices of the brain cavity. The dosimetric calculations, carried out as a first step according to a beta-emitter radiation model with a spherical geometry and, as a second step, according to a flat geometry model, have sown that only within the first 3-5 millimetres of the tissue surrounding the cavity the dose deposition is sufficient to destroy the neoplastic cells left after the exeresis.

A dosimetry example for a patient suffering from glioblastoma, wherein the internal surface area of the cavity was evaluated as being about 12 cm², treated with three subsequent administrations of the radiopharmaceutical product according to the invention, is shown in the following table. The first administration consisted in 2 mCi of yttrium-90 prepared as described in example 1, the second administration was 5 mCi of yttrium-90 prepared as before and the third administration consisted in 15 mCi of holmium-166 prepared according to the procedure described in Example 2.

**TABLE Absorbed dose as a function of the depth from the cavity margin**

| Distance, mm | ⁹⁰Y, 2 mCi Gy | ⁹⁰Y, 5 mCi Gy | ¹⁶⁶Ho, 15 mCi Gy | Total Gy |
|---|---|---|---|---|
| 1 | **250** | **620** | **680** | **1550** |
| 2 | **140** | **360** | **390** | **890** |
| 3 | **80** | **190** | **210** | **480** |
| 4 | 36 | **90** | **100** | **226** |
| 5 | 18 | *45* | *46* | **109** |
| 6 | 7 | 18 | 20 | *45* |
| 7 | 3 | 8 | 9 | 20 |

The preceding table shows in bold face type the lethal doses, in italic type the sub-lethal doses and in normal type the non-lethal doses for living cells. From the above table it may be observed that the treatment with the preparation according to the invention was effective in eliminating the cancerous cells present within a margin of 5-6 mm from the edge of the cavity, but did not appreciably affect the cells placed at a higher distance. The examination of the same patient by scintigraphy confirmed that the radioactivity remains confined within the brain cavity, where it is detectable up to the complete decay of the isotope, with no appreciable radioactivity leaks towards the remainder of the body.

As noted in the foregoing, preparations similar to the ones described in the examples can be used in brachytherapy for the treatment of other forms of cancer, both by intracavitary administration and by intratissutal or intraarterial administration. Besides the treatment of hepatic tumor lesions, both primary and from metastasis, as referred to in the foregoing, the product may be employed, e.g., for the treatment of hypophysis adenomas, that are benign tumors affecting the hypophysis gland. Although slowly growing, such formations occupy a space within the bony skull and may exert a dangerous pressure on the optic chiasma. As consequence, hypophysis adenomas are normally removed by surgery. Also in this case a regrowth by contiguity is possible, leading to the need of periodically repeating the removal operations. By using the gel with radioactive microparticles according to the invention, it is possible to insert the said gel as a thin layer in the surgical cavity (appropriately dosing the products so that the radiation is not allowed to damage the neighbouring neural structures). Then, the cavity (which is accessible for surgery from the nasal duct) may be sealed with a layer of fibrin, in order to avoid any leaks of the gel.

Another field wherein the product of the invention may advantageously be used is, as pointed out before, the treatment of patients suffering from hypersplenism of various origin. Hypersplenisms consists in an abnormal swelling of the spleen, with simultaneous depletion of one or more types of blood cells. The said syndrome may be caused by different haematological, neoplastic and inflammatory diseases, or by portal hypertension. In particular, hypersplenism affects children suffering from Cooley's anaemia or thalassemia major (also known as Mediterranean anaemia), a disease for which practically no cure exists at present, which is characterised by an extremely short life of red blood cells and by splenomegaly. This disease is treated with repeated transfusions. The rapid destruction of red blood cells carried out by the spleen frees large amounts of iron, that accumulate in the body, exposing the patient to a remarkable heart and liver overload. For the treatment of these patients, as well as in other cases of hypersplenism where a splenectomy is not advisable (it should be noted that the spleen has an important function in the immune system) radioembolization interventions by intraarterial administration (i.e. through a catheter placed in the splenic artery) of beta-emitting radioisotopes have been proposed. In particular, Becker et al. (C.D. Becker, H. Rösler, F. Demarmels Biasiutti, H.U. Bauer, Radiology, 195, 183-186, (1995)) report on a case of splenic radioembolization for the treatment of a patient with congestive hypersplenism, employing resin microparticles loaded with ⁹⁰Y. The microparticles were injected in the splenic artery suspended in an aqueous solution of glucose and dextran.

It is evident that the radioactive product with bioabsorbable microparticles in a gel matrix according to the invention may advantageously be used in place of the product described in the prior art cited above. The advantage is due both to the complete absorbability of the carrier particles and, especially, to the features of the gel medium, that allows an accurate dosage in the injection and a uniform dose distribution in the tissue.

A further field of application of the preparation according to the invention concerns the use as a "flag" in radio-guided surgery, in the treatment of breast cancer. In many cases, small sized tumor lesions are visible upon echographic or mammographic examination, but at the moment of the exeresis they escape the visual perception of the surgeon. In order to overcome this problem it is customary to "label" the tumor lesions at the moment of the echographic examination by means of the insertion of a metallic wire (i.e. a "flag") in the tumor. In this way, the surgeon may readily locate any tumor mass to be excised. Besides being painful for the patient, this method is often quite unpractical for the surgeon, as the metallic wire may be difficult to locate during the surgical operation. In addition, the metallic wire may be displaced from its original site. An example of a preparation according to the invention for use in the flagging of breast tumors is described below.

### EXAMPLE 3

A kit for use in radio-guided surgery for the removal of breast carcinoma formations consisted of three test-tubes. The first test-tube contained 5 ml of a gel analogous to the one described in Example 1. The second test-tube contained 150 mg of AG1x8 Dowex resin and 0.15 ml of water, and the third test-tube contained a solution of ¹³¹I carrier-free, having an activity of about 15 µCi. The contents of all test-tubes was sterile, as a result of a treatment in autoclave at 121 °C for 15 minutes.

The method of use of the above kit is as follows:
a) the ¹³¹I contents of the third test-tube, in a minimum volume of water (typically, 1 ml), is introduced, by means of a sterile syringe equipped with a 0.22 µm filter, in the test-tube containing the resin;
b) the mixture is stirred for about 1 hour at room temperature;
e) the procedure is continued as in items b)-e) of Example 1.

In this case, the use of the product according to the invention, injected in the tumor lesions in stereotaxy and under echographic or radiographic visualisation, allows to eliminate any inconvenience to the patient and makes simpler and safer the subsequent surgical operation, since the radioisotope-labelled neoplastic formation may readily be detected by the surgeon with the aid of a collimated radioactivity probe of the kind already on the market for radio-guided surgery. The gel preparation according to the invention insures that that every drop expelled by the syringe in the stage of "flagging" the tumor lesions contains a controllable and uniform amount of radioactive particles. On the other hand, in this case it is not necessary that the microspheres making the radioisotope carrier be bioabsorbable, as the formations containing the product of the invention are bound to be quite soon removed.

## Claims

1. A radioactive composition for use in therapy and as a surgical aid, containing radioisotopes immobilized on biocompatible or bioabsorbable solid particles obtainable from separately produced solid particles, on which the said radioisotopes are subsequently immobilized by chelation, or obtainable from particles that already incorporate the non-radioactive chemical element corresponding to the desired radioisotope and which particles are then irradiated in a nuclear reactor under neutron flux to obtain the said radioisotopes,
**characterised in that** the said solid particles are incorporated in a biocompatible and bioabsorbable matrix consisting of a hypotonic gel containing, in water, from 2 to 30% by weight of polyvinylpyrrolidone and from 0.01 to 2% by weight of agar-agar or agarose.

2. A composition according to claim 1, wherein the average molecular weight (MW) of said polyvinylpyrrolidone (PVP) is in the range from 50,000 to 400,000.

3. A composition according to claim 2, wherein the said gel has the following formulation (the percentages being by weight):
• PVP with average MW 340,000-380,000 15-25%
• purified agar-agar 0.1-0.2%
• deionised sterile water q.s. to 100%.

4. A composition according to any one of claims 1-3, wherein the said solid particles are chosen among bioinert particles made of ion exchange resins and bioabsorbable particles made of poly(lactic acid).

5. A composition according to any one of claims 1-4, wherein the said particles have diameters in the range from 0.01 µm to 100 µm.

6. A composition according to any one of the previous claims, wherein the said radioisotopes are beta- and/or gamma-emitting radioisotopes.

7. A composition according to claim 6, wherein the said radioisotopes are beta-emitting radioisotopes with energy > 0,3 MeV.

8. A composition according to claim 6, wherein the said radioisotopes are chosen from ⁹⁰Y, ¹⁶⁶Ho, ¹³¹I, ¹⁸⁶Re and ¹⁸⁸Re.

9. A radiopharmaceutical kit or a kit for use in radio-guided surgery comprising a first component containing biocompatible or bioabsorbable solid particles chosen among bioinert particles made of ion exchange resins and bioabsorbable particles made of poly(lactic acid), and a second component consisting of a hypotonic gel containing, in water, from 2 to 30% by weight of polyvinylpyrrolidone and from 0.01 to 2% by weight of agar-agar or agarose.

10. A kit according to claim 9 further containing a third component consisting of beta- and/or gamma-emitting radioisotopes.

11. A kit according to claim 9 wherein the said biocompatible or bioabsorbable solid particles of said first component already incorporate the non-radioactive chemical element corresponding to the desired radioisotope.

12. A kit according to any one of claims 9-11, wherein the average molecular weight (MW) of said polyvinylpyrrolidone (PVP) is in the range from 50,000 to 400,000.

13. A kit according to claim 12, wherein the said gel has the following formulation (the percentages being by weight):
• PVP with average MW 340,000-380,000 15-25%
• purified agar-agar 0.1-0.2%
• deionised sterile water q.s. to 100%.

## Patentansprüche

1. Radioaktive Zusammensetzung zur Verwendung in einer Therapie und als chirurgisches Hilfsmittel, die Radioisotope enthält, die auf biokompatiblen oder bioabsorbierbaren festen Partikeln immobilisiert sind, die erhältlich sind aus getrennt hergestellten festen Partikeln, auf denen die Radio-isotope durch Chelatbildung nacheinander immobilisiert werden, oder die erhältlich sind aus Partikeln, die das nicht-radioaktive chemische Element inkorporieren, das dem gewünschten Radioisotop entspricht und wobei diese Partikel dann unter Neutronenfluss in einen Kernreaktor eingestrahlt werden, um die Radioisotope zu erhalten, **dadurch gekennzeichnet, dass** die festen Partikel in eine biokompatible und bioabsorbierbare Matrix inkorporiert sind, die aus einem hypotonischen Gel besteht, das in Wasser zwischen 2 und 30 Gewichtsprozent Polyvinylpyrrolidon und zwischen 0,01 und 2 Gewichtsprozent Agar-Agar oder Agarose enthält.

2. Zusammensetzung gemäß Anspruch 1, wobei das durchschnittliche Molekülgewicht (MW) des Polyvinylpyrrolidon (PVP) im Bereich zwischen 50.000 und 400.000 liegt.

3. Zusammensetzung gemäß Anspruch 2, wobei das Gel die folgende Formulierung hat (wobei die Prozente das Gewicht betreffen):
- PVP mit einem durchschnittlichen MW 340.000 - 380.000: 15 - 25 %
- gereinigtes Agar-Agar: 0,1 - 0,2 %
- entionisiertes steriles Wasser: q.s. bis 100 %

4. Zusammensetzung nach einem der Ansprüche 1 - 3, wobei die festen Partikel aus bioinerten Partikeln ausgewählt sind, die aus Ionenaustauschharzen und bioabsorbierbaren Partikeln hergestellt sind, die aus Poly (-Milchsäure) hergestellt sind.

5. Zusammensetzung nach einem der Ansprüche 1 - 4, wobei die Partikel Durchmesser im Bereich von 0,01 µm bis 100 µm haben.

6. Zusammensetzung nach einem der vorangehenden Ansprüche, wobei die Radioisotope beta- und/oder gamma-emittierende Radioisotope sind.

7. Zusammensetzung gemäß Anspruch 6, wobei die Radioisotope beta-emittierende Radioisotope einer Energie > 0,3 MeV sind.

8. Zusammensetzung gemäß Anspruch 6, wobei die Radioisotope ausgewählt sind aus ⁹⁰Y, ¹⁶⁶Ho, ¹³¹I, ¹⁸⁶Re und ¹⁸⁸Re.

9. Radiopharmazeutische Ausrüstung oder Ausrüstung zur Verwendung bei der strahlungsgeführten Chirurgie, umfassend eine erste Komponente, die biokompatible oder bioabsorbierbare feste Partikel enthält, die aus bioinerten Partikeln ausgewählt sind, die aus Ionenaustauschharzen und bioabsorbierbaren, aus Poly (-Milchsäure) hergestellten Partikeln bestehen, und eine zweite Komponente, die aus einem hypotonischen Gel besteht, das in Wasser von 2 - 30 Gewichtsprozent Polyvinylpyrrolidon und von 0,01 - 2 Gewichtsprozent von Agar-Agar oder Agarose enthält.

10. Ausrüstung gemäß Anspruch 9, weiterhin enthaltend eine dritte Komponente, die aus beta- und/oder gamma-emittierenden Radioisotopen besteht.

11. Ausrüstung gemäß Anspruch 9, wobei die biokompatiblen oder bioabsorbierbaren festen Partikel der ersten Komonente schon das nicht-radioaktive, chemische Element inkorporieren, das dem gewünschten Radioisotop entspricht.

12. Ausrüstung nach einem der Ansprüche 9 - 11, wobei das durchschnittliche Molekülgewicht (MW) des Polyvinylpyrrolidons (PVP) im Bereich von 50.000 - 400.000 liegt.

13. Ausrüstung gemäß Anspruch 12, wobei das Gel die folgende Formulierung hat (die Prozente betreffen das Gewicht):
- PVP mit einem durchschnittlichen MW 340.000 - 380.000: 15 - 25 %
- gereinigtes Agar-Agar: 0,1 - 0,2 %
- entionisiertes steriles Wasser: q.s. bis 100 %.

## Revendications

1. Composition radioactive destinée à être utilisée dans une thérapie et en tant qu'aide chirurgicale, contenant des radio-isotopes immobilisés sur des particules solides biocompatibles ou bioabsorbables pouvant être obtenus à partir de particules solides produites séparément, selon laquelle lesdits radio-isotopes sont ultérieurement immobilisés par chélation ou peuvent être obtenus à partir de particules qui incorporent déjà l'élément chimique non radioactif correspondant au radio-isotope souhaité, et lesquelles particules sont ensuite irradiées dans un réacteur nucléaire sous un flux de neutrons pour obtenir lesdits radio-isotopes,
**caractérisé en ce que** lesdites particules solides sont intégrées dans une matrice biocompatible et bioabsorbable consistant en un gel hypotonique contenant, dans de l'eau, entre 2 et 30 % en poids de polyvinylpyrrolidone et entre 0,01 et 2 % en poids d'agar-agar ou d'agarose.

2. Composition selon la revendication 1, dans laquelle le poids moléculaire moyen (MW) de ladite polyvinylpyrrolidone (PVP) se trouve dans la plage comprise entre 50 000 et 400 000.

3. Composition selon la revendication 2, dans laquelle ledit gel a la formulation suivante (les pourcentages étant donnés en poids) :
• PVP avec un MW moyen de 340 000 - 15 et 25 % 380 000
• Agar-agar purifié 0,1 à 0,2 %
• Eau stérile déminéralisée q.s. jusqu'à 100 %

4. Composition selon l'une quelconque des revendications 1 à 3, dans laquelle lesdites particules solides sont choisies parmi des particules bio-inertes composées de résines échangeuses d'ions et de particules bioabsorbables composées d'acide polylactique.

5. Composition selon l'une quelconque des revendications 1 à 4, dans laquelle lesdites particules ont un diamètre dans la plage comprise entre 0,01 µm et 100 µm.

6. Composition selon l'une quelconque des revendications précédentes, dans laquelle lesdits radio-isotopes sont des radio-isotopes émetteurs de rayonnement bêta et/ou gamma.

7. Composition selon la revendication 6, dans laquelle lesdits radio-isotopes sont des radio-isotopes émetteurs de rayonnement bêta avec une énergie > 0,3 MeV.

8. Composition selon la revendication 6, dans laquelle lesdits radio-isotopes sont choisis parmi ⁹⁰Y, ¹⁶⁶Ho, ¹³¹I, ¹⁸⁶Re et ¹⁸⁸Re.

9. Kit radiopharmaceutique ou kit destiné à être utilisé en chirurgie radioguidée comprenant un premier composant contenant des particules solides biocompatibles ou bioabsorbables choisies parmi les particules bio-inertes composées de résines échangeuses d'ions et de particules bioabsorbables composées d'acide polylactique, et un deuxième composant consistant en un gel hypotonique contenant, dans de l'eau, entre 2 et 30 % en poids de polyvinylpyrrolidone et entre 0,01 et 2 % en poids d'agar-agar ou d'agarose.

10. Kit selon la revendication 9 contenant en outre un troisième composant consistant en des radio-isotopes émetteurs de rayonnement bêta et/ou gamma.

11. Kit selon la revendication 9, dans lequel lesdites particules solides biocompatibles ou bioabsorbables dudit premier composant intègrent déjà l'élément chimique non radioactif correspondant au radio-isotope souhaité.

12. Kit selon l'une quelconque des revendications 9 à 11, dans lequel le poids moléculaire moyen (MW) de ladite polyvinylpyrrolidone (PVP) est dans la plage comprise entre 50 000 et 400 000.

13. Kit selon la revendication 12, dans lequel ledit gel a la formulation suivante (les pourcentages étant donnés en poids) :
• PVP avec MW moyen de 340 000 15 à 25 % à 380 000
• Agar-agar purifié 0,1 à 0,2 %
• Eau stérile déminéralisée q.s. jusqu'à 100 %
